# EUROPÄISCHE PATENTSCHRIFT

(11) **EP 1 402 855 B1**
(45) Veröffentlichungstag und Bekanntmachung des Hinweises auf die Patenterteilung: **01.09.2004**
(21) Anmeldenummer: 02021384.9
(22) Anmeldetag: 24.09.2002
(51) Int. Cl.: A61F 2/46, A61B 19/00

(54) **Vorrichtung und Verfahren zum Bestimmen des Öffnungswinkels eines Gelenks**
Device and method for determining the flexion angle of a joint
Appareil et méthode pour déterminer le débattement d'une articulation

(43) Veröffentlichungstag der Anmeldung: 31.03.2004
(73) Patentinhaber: BrainLAB AG, 85551 Kirchheim/Heimstetten (DE)
(72) Erfinder: Tuma, Gregor, 81675 München (DE); Schubert, Mario, 85652 Landsham (DE); Schaffrath, Claus, Dr., 81377 München (DE)
(74) Vertreter: Schwabe - Sandmair - Marx

(56) Entgegenhaltungen:
- WO-A-02/02028
- US-A- 4 804 000
- US-A- 5 197 488
- US-A- 6 002 859
- US-A1- 2002 107 522

## Beschreibung

Die vorliegende Erfindung bezieht sich auf eine Vorrichtung und ein Verfahren zum Bestimmen des Öffnungswinkels eines Gelenks. Der nächstliegende Stand der Technik ist US-A-4 804 000.

Insbesondere bezieht sich die Erfindung auf die Bestimmung eines Öffnungswinkels eines Gelenks, welches zwischen zwei Körperstrukturen liegt, wie zum Beispiel den Öffnungswinkel eines Hüftgelenks, eines Kniegelenks, eines Ellenbogengelenks oder anderer Gelenke, wobei zum Beispiel im Falle des kugelgelenkähnlichen Hüftgelenks unter dem Begriff "Öff nungswinkel" sowohl der Winkel zwischen einer festgelegten Oberschenkelachse und einer durch die Lage der Hüfte definierten Ebene oder Achse, als auch die räumliche Lage oder Orientierung des Winkels verstanden werden soll. Es soll z. B. ermittelt werden wie weit der Oberschenkelknochen relativ zur Hüfte zum Beispiel nach vorne, nach hinten oder zur Seite verkippt ist, d.h. der Raumwinkel welchen das Gelenk bildet wird bestimmt. Ebenso soll z.B. ermittelt werden wie ein Gelenk oder Teile oder Strukturen davon beispielsweise um eine Mittelachse gedreht sind.

Künstliche Hüftgelenke sind bekannt, bei welchen ein Femur-Implantat in einen körpereigenen Oberschenkelknochen (Femur) nach Entfernung eines körpereigenen Gelenkkopfes eingebracht wird. Als Gegenlager wird in die Hüfte eine Gelenkpfanne eingesetzt, welche als Wiederlager einen sphärischen oder teilsphärischen Kopf des Femur-Implantats aufnehmen kann. Der genaue Sitz der Implantate ist ein wesentliches Kriterium für den Erfolg einer Hüftgelenkimplantation, wobei schon kleinste Abweichungen zu einem überdurchschnittlich schnellen Verschleiß und damit zu einer geringen Lebensdauer des Hüftgelenkimplantats führen. Insbesondere bei plötzlich auftretenden Belastungen, wie zum Beispiel Stößen, kann ein nicht genau eingesetztes Hüftimplantat leicht ausgerenkt werden oder sich lockern, so dass es vorteilhaft ist, bereits intra-operativ den richtigen Sitz und die korrekte Positionierung eines implantierten Gelenks zu überprüfen.

Es ist bekannt, dass ein Chirurg manuell während einer Hüftgelenkimplantation das Bein eines Patienten in verschiedene Richtungen bewegt bis die weitere Bewegung durch die Anatomie des Patienten, durch ein Aufeinanderstoßen der implantierten Komponenten und angrenzender Knochen oder aufgrund von Behinderungen durch Gewebe oder Bänder verhindert wird. Durch eine solche manuelle Bewegung beurteilt ein erfahrener Chirurg die Beweglichkeit des Gelenks und somit den richtigen Sitz der einzelnen Komponenten eines implantierten Gelenks. Jedoch kann ein Chirurg so nur sehr grob abschätzen, ob die so ermittelte und abgeschätzte Beweglichkeit für das tägliche Leben eines Patienten ausreichend ist.

Aus der US 6,002,859 sind eine Vorrichtung und ein Verfahren zum Bestimmen der Position einer Komponente eines Implantats bekannt, wobei ein Modell des Gelenks und der Implantat-Komponenten erzeugt werden und eine Bewegung des Gelenks anhand der Modelle simuliert wird.

Es ist eine Aufgabe der vorliegenden Erfindung eine Vorrichtung und ein Verfahren zum Bestimmen des Öffnungswinkels eines Gelenks vorzuschlagen, welche zur genauen Überprüfung des richtigen Sitzes von Implantat-Komponenten verwendet werden können.

Diese Aufgabe wird durch die Gegenstände der unabhängigen Ansprüche gelöst.

Vorteilhafte Ausführungsformen ergeben sich aus den Unteransprüchen.

Die erfindungsgemäße Vorrichtung zum Bestimmen des Öffnungswinkels eines Gelenks, wie zum Beispiel eines natürlichen oder eines künstlichen Gelenks, und/oder zum Bestimmen von Relativpositionen von Komponenten oder Strukturen, kann sowohl vor, während, als auch nach der Implantation eines Gelenkimplantats verwendet werden, um zum Beispiel intra-operativ den Öffnungswinkel oder den Bewegungsbereich (range of motion) eines Gelenks zu ermitteln und falls erforderlich Modifikationen an den implantierten Gelenk-Komponenten oder der Position einer Gelenk-Komponente und/oder an der Patientenanatomie, z.B. durch chirurgische Eingriffe durchführen zu können. Es ist eine Erfassungsvorrichtung, wie zum Beispiel eine Kamera zur Erfassung von sichtbarem oder Infrarot-Licht, elektromagnetische Sensoren ("Magnettracking"), ein Schall-Sensor, oder ein auf Funk basierendes System vorgesehen, mit welcher die Position der das Gelenk bildenden Komponenten und/oder der mit dem Gelenk verbundenen oder zu verbindenden Strukturen ermittelt werden kann. Vorteilhaft werden Veränderungen der Position und/oder der Drehlage einer oder mehrerer Komponenten oder Strukturen erfasst, so dass z. B. die Veränderungen in sechs Freiheitsgraden ermittelt werden können. Dabei können auf bekannte Weise zum Beispiel Marker an den jeweiligen das Gelenk bildenden Komponenten oder angrenzenden Strukturen, wie zum Beispiel an der Hüfte und dem Oberschenkelknochen und/oder an den implantierten Komponenten angebracht werden, um nach Registrierung der entsprechenden zum Beispiel mit Markern versehenen Elemente die Position der jeweiligen Strukturen oder Komponenten zu erfassen. Weiterhin ist eine Recheneinheit vorgesehen, welcher die von der Erfassungsvorrichtung ermittelten Positionssignale, wie zum Beispiel die optisch erfassten Umrisse einer Struktur oder zum Beispiel von Markem ausgesandte Signale und damit die Position der mit diesen Markern verbundenen Elemente oder Strukturen zugeführt wird, um erfindungsgemäß aus diesen Informationen den Öffnungswinkel oder allgemein den Raumwinkel eines Gelenks bzw. eines durch zwei Strukturen gebildeten Winkels zu ermitteln.

Durch die erfindungsgemäße Vorrichtung wird somit eine Verbesserung der Überprüfung und Beurteilung und damit eine verbesserte Durchführung einer Implantation eines künstlichen Gelenks ermöglicht, so dass das Ergebnis der Implantation verbessert werden kann. Es können automatisch Positionswerte und Winkelwerte ermittelt werden, um zum Beispiel anhand von manuell oder automatisch, zum Beispiel unter Verwendung von Robotern, durchgeführten Bewegungen eine Überprüfung des richtigen Sitzes eines Gelenks durchzuführen. Dabei kann zum Beispiel unter Berücksichtigung von anatomisch relevanten Einflussparametem, wie z.B. der Bänder, Weichteile, etc. ermittelt werden, ob die mit dem implantierten Gelenk durchführbaren Bewegungen ausreichend sind, oder ob das Implantat oder der Sitz des Implantates noch verändert werden müssen, um den gewünschten Bewegungsbereich des Gelenkes zu erhalten. Zum Beispiel kann ein Gelenk in jede Richtung soweit bewegt werden, bis es durch einen Zusammenstoß mit einer Körperstruktur, mit einer Komponente des Gelenks oder durch andere Faktoren bei normalem Kraftaufwand nicht mehr möglich ist, das Gelenk weiter zu öffnen oder zu schließen, so dass der Bewegungsbereich des Gelenkes in verschiedene Richtungen ermittelt werden kann. Dabei können durch die erfindungsgemäße Vorrichtung genaue Winkelwerte ermittelt werden, welche zum Beispiel mit vorgegebenen Referenzwerten verglichen werden können, um den richtigen Sitz oder die korrekte Funktionsweise eines Gelenks zu überprüfen, ohne dabei auf die Erfahrung eines Chirurgen angewiesen zu sein.

Erfindungsgemäß können somit auch intra-operativ die tatsächlichen Positionen der Gelenk-Komponenten und/oder der mit dem Gelenk verbundenen Körperstrukturen, wie zum Beispiel der Hüfte und des Oberschenkelknochens, im dreidimensionalen Raum ermittelt und damit die relative Lage zueinander bestimmt werden, so dass präzise der Öffnungswinkel des Gelenks in verschiedenen Richtungen oder die die Lage und Orientierung im Raum bestimmenden sechs Freiheitsgrade genau gemessen werden können.

Vorteilhaft ist eine Datenausgabevorrichtung, wie zum Beispiel eine Anzeige zur Ausgabe des ermittelten Öffnungswinkels des Gelenks oder ein Bildschirm vorgesehen, auf welchem zum Beispiel die dreidimensionale Lage des Gelenks im Raum zusammen mit den die Stellung des Gelenks beschreibenden Parametern, wie zum Beispiel dem Öffnungswinkel und der Lage des Öffnungswinkels im Raum dargestellt werden kann.

Gemäß einer bevorzugten Ausführungsform ist eine Vorrichtung zum definierten Anlegen von Kräften in definierten Richtungen an das Gelenk bzw. an eine bestimmte Gelenk-Komponente vorgesehen, wie zum Beispiel ein Roboter oder eine manuell betätigbare Vorrichtung, welche eine angelegte Kraft anzeigen kann, um ermitteln zu können wie weit ein Gelenk bei bestimmten auftretenden Kräften bewegt wird, um definierte Messergebnisse zu erhalten, welche zum Beispiel mit vorher aufgenommenen Referenzwerten verglichen werden können.

Nach dem erfindungsgemäßen Verfahren wird der Öffnungswinkel eines Gelenks bestimmt, indem die Position der das Gelenk bildenden Strukturen erfasst wird und aus den erfassten Positionen erfindungsgemäß der Öffnungswinkel ermittelt wird. Das Verfahren kann zum Beispiel intra-operativ verwendet werden, um zum Beispiel während oder nach einem chirurgischen Eingriff den Öffnungswinkel oder allgemein die Bewegungsmöglichkeit eines Gelenks zu messen, um somit Informationen zu liefern, welche die Arbeit zum Beispiel eines Chirurgen unterstützen. Zur Durchführung des Verfahrens ist kein chirurgischer Eingriff erforderlich, da lediglich die Position der das Gelenk bildenden Komponenten und/oder der angrenzenden Körperstrukturen zum Beispiel durch daran angebrachte Marker, durch Erfassung der Umrisse oder durch andere geeignete Verfahren aufgenommen wird und aus den so aufgenommenen Informationen der Öffnungswinkel berechnet wird.

Vorteilhaft werden zur Bestimmung des Öffnungswinkels oder Raumwinkels eines Gelenks Aufnahmen des natürlichen oder künstlichen Gelenks und/oder der an das Gelenk angrenzenden Körperstrukturen verwendet, wobei zum Beispiel Kernspinresonanz(MR)-, Computertomographie(CT)- , Ultraschall- oder andere geeignete Verfahren verwendet werden können. Die aufgenommenen Körperstrukturen können zum Beispiel durch bekannte Segmentationsoder Trennverfahren in einzelne Elemente unterteilt werden, so dass aus den aufgenommenen Daten die Grenzen von aneinander angrenzenden Strukturen erhalten werden können, um zum Beispiel Daten zur Berechnung des Öffnungswinkels aus Positionsdaten der Körperstrukturen oder zur Berechnung einer optimalen Position eines zu implantierenden Gelenks zu haben. Allgemein sind bei einem Bilddatensatz die Knochenstrukturen in einer Art und Weise erfasst, welche im Wesentlichen davon abhängig ist, wie der Patient zum Zeitpunkt der Bilddatenerfassung positioniert bzw. gelegen ist. Es gibt zwar grobe Anweisungen an die Radiologie und an den Patienten bezüglich einer Soll-Lage zur Aufnahme des Bilddatensatzes, jedoch müssen die variablen Positionen der einzelnen Knochenstrukturen virtuell auf eine zu definierende Ausgangsposition oder Neutralposition gebracht werden, um eine genau Vergleichsbasis zu schaffen, welche auch einen Vergleich zwischen mehreren Patienten ermöglicht. Es können z. B. drei lokale Koordinatensystem nach der Segmentierung definiert werden, wie z.B. Femur links, Femur rechts und Becken, welche anschließend gemäß einer festgelegten Vorschrift zueinander ausgerichtet und somit in ein definiertes Lageverhältnis gebracht werden. Will man jetzt Positionsdaten, wie z.B. Winkel, Beinlänge, usw. eines Gelenks visualisieren, so kann diese Neutralposition als Startposition verwendet werden, bezüglich welcher ein Öffnungswinkel oder eine räumliche Lage definiert wird. Somit können auch bei verschiedenen Patienten reproduzierbare und untereinander vergleichbare Ausgangsbedingungen geschaffen werden.

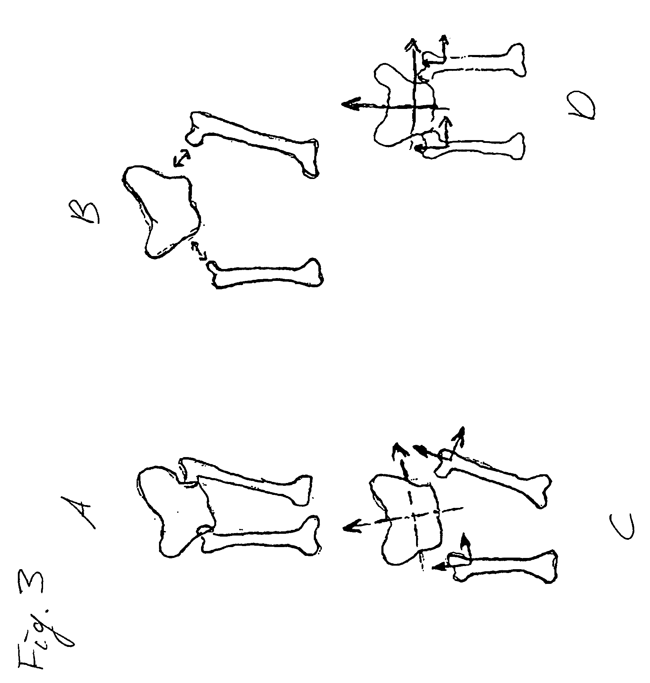

Figur 3 zeigt prinzipiell die oben beschriebene Vorgehensweise, wobei in Figur 3A der in einer beliebigen Lage des Patienten aufgenommene Bilddatensatz schematisch gezeigt ist. Durch eine Segmentierung, wie schematisch in Fig. 3B gezeigt, werden in dem Ausführungbeispiel die einzelnen Knochenstrukturen, Hüfte, Femur links und Femur rechts als einzelne Elemente erkannt. In Figur 3C werden den einzelnen segmentierten Strukturen Koordinatensysteme zugeordnet, welche die virtuelle Ausrichtung in der in Figur 3D gezeigten Neutralposition ermöglichen.

Bevorzugt werden an dem zu implantierenden Gelenk, insbesondere an den einzelnen Komponenten des Gelenks und/oder an den an das Gelenk angrenzenden Körperstrukturen Referenzelemente, wie zum Beispiel reflektierende Marker angebracht, um die räumliche Lage des Gelenks bzw. der einzelnen Gelenk-Komponenten und/oder der entsprechenden Körperstrukturen nach einer Registrierung der jeweiligen Elemente ermitteln und verfolgen zu können, so dass zum Beispiel zur Ermittlung eines möglichen Bewegungsbereichs eines Gelenks die räumlichen Positionen der einzelnen Gelenk-Komponenten und/oder die räumlichen Positionen der an das Gelenk angrenzenden Körperstruktur zur Verfügung stehen.

Vorteilhaft werden die Parameter, welche die räumliche Lage und den Öffnungswinkel des Gelenks angeben, visualisiert, so dass bevorzugt in Echtzeit gesehen werden kann, welche Winkelstellung momentan vorliegt.

Gemäß einer bevorzugten Ausführungsform werden Daten, welche die Bewegungsmöglichkeit eines natürlichen oder implantierten Gelenks beschreiben, gemessen und gespeichert, so dass mindestens ein Referenzwert zur Verfügung steht, um ein neu implantiertes Gelenk auf den richtigen Sitz und die gewünschten Bewegungsmöglichkeiten überprüfen zu können. Eine Überprüfung eines implantierten Gelenks kann zum Beispiel durch einen Vergleich der gemessenen Bewegungsmöglichkeiten des implantierten Gelenks mit Messdaten von natürlichen, also nicht implantierten Gelenken, oder mit erfolgreich implantierten Gelenken durchgeführt werden. Ebenso können z. B. bei einem Hüftgelenk aufgrund der Symmetrie Vergleichsdaten eines dem zu ersetzenden Gelenks gegenüberliegenden Gelenks verwendet werden, um Referenzdaten für die Positionierung eines zu implantierenden Gelenks zu erhalten.

Bevorzugt werden an das Gelenk definierte Kräfte in definierten Richtungen angelegt, um Vergleichswerte bei definierten Belastungen des Gelenks zu haben, wodurch eine Überprüfung eines implantierten Gelenks durchgeführt werden kann.

Gemäß einem weiteren Aspekt bezieht sich die vorliegende Erfindung auf ein Computerprogramm, welches wenn es in einen Computer geladen ist oder auf einem Computer läuft, mindestens einen der oben beschriebenen Verfahrensschritte durchführt. Nach einem weiteren Aspekt bezieht sich die Erfindung auf ein Speichermedium für ein Programm oder ein Computerprogrammprodukt, welches ein solches Programm aufweist.

Die Erfindung wird nachfolgend anhand einer bevorzugten Ausführungsform beschrieben werden, wobei:
- Fig. 1: schematisch eine erfindungsgemäße Vorrichtung zeigt;
- Fig. 2: ein Ablaufdiagramm einer Ausführungsform des erfindungsgemäßen Verfahrens zeigt; und
- Fig. 3A-D: schematisch ein Verfahren zum Bestimmen einer Neutralposition zeigt.

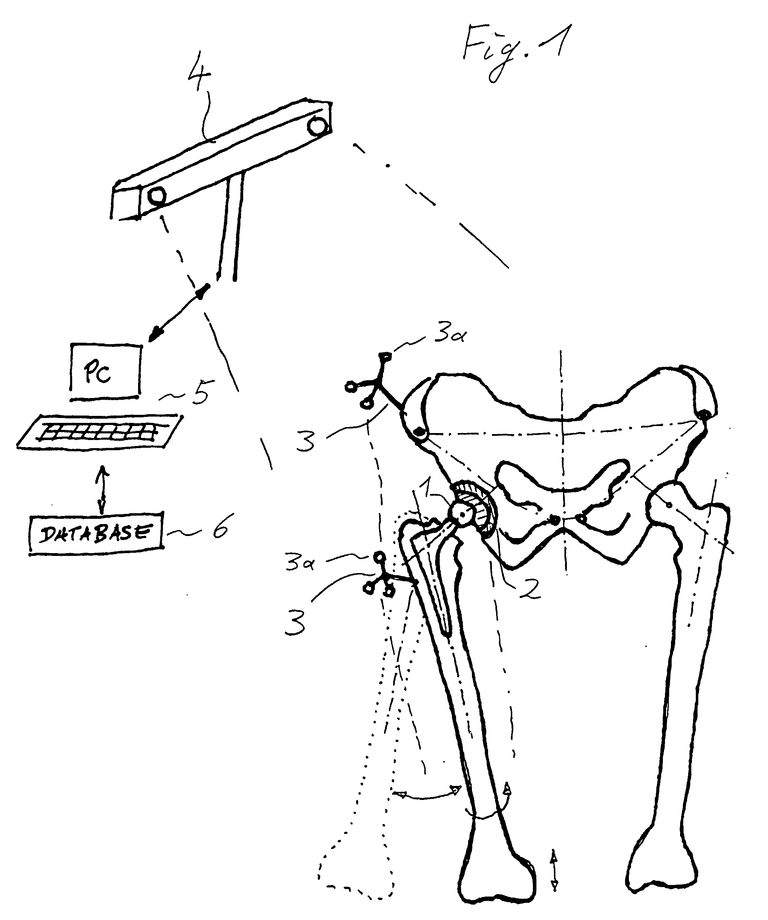

Figur 1 zeigt schematisch ein künstliches Hüftgelenk mit einem Gelenkkopf 1 eines Femur-Implantats, welcher von einer Gelenkpfanne 2 der Hüfte aufgenommen wird. An den mit dem Gelenkkopf 1 und der Gelenkpfanne 2 verbundenen Körperstrukturen und/oder an dem Gelenkkopf 1 und/oder der Gelenkpfanne 2 selbst sind jeweils Referenzsterne 3 mit Markern 3a angebracht, um die Position des Gelenkkopfes 1 und der Gelenkpfanne 2 bestimmen zu können. Eine IR-Kamera 4 nimmt von den Markem 3a der Referenzsterne 3 reflektiertes Licht auf und gibt die Signale an eine Recheneinheit 5 weiter. Die Recheneinheit 5 kann die Position und/oder den Raumwinkel in den orthopädisch definierten Richtungen Abduktion, Adduktion, Flexion, Extension, interne und externe Rotation und Beinlängenveränderung anzeigen. Es ist auch möglich einen Winkel zwischen einer vorher definierten Achse durch den Gelenkkopf 1 und einer vorher definierten Achse durch die Gelenkpfanne 2 zu berechnen. Zur Berechnung des Winkels kann z. B. auf in einer Datenbank 6 gespeicherte Daten zurückgegriffen werden, welche beispielsweise Informationen zur Geometrie des Gelenkkopfes 1, der Gelenkpfanne 2, der Befestigungsposition der Referenzsterne 3 oder andere Informationen enthält. Weiterhin können in der Datenbank 6 zum Beispiel Referenzwerte einer durchgeführten Mess-Serie abgespeichert werden, welche zum Vergleich mit nachfolgenden Messungen verwendet werden können.

Als Referenzsystem kann z. B. ein Koordinatensystem des Femurs verwendet werden, welches definiert ist durch die Ebene, welche durch die Halsachse (Mittelachse des Schenkelhalses) und die Schaftachse (Mittelachse des Femur-Röhrenknochens) gebildet wird. Diese Ebene wird um den Femur-Rotationspunkt parallel zur frontalen Beckenebene ausgerichtet.

Als Vergleichsreferenz, z. B. für den zu ermöglichenden Bewegungsbereich oder für die Beinlänge kann das gesunde dem zu implantierenden Gelenk gegenüberliegende Gelenk oder Bein verwendet werden.

Der von der Recheneinheit 5 ermittelte Raumwinkel kann an eine Anzeigeeinheit, wie zum Beispiel einen Bildschirm, ausgegeben werden und dort numerisch, als graphische Darstellung des Gelenks und/oder mit Zusatzinformationen ausgegeben werden.

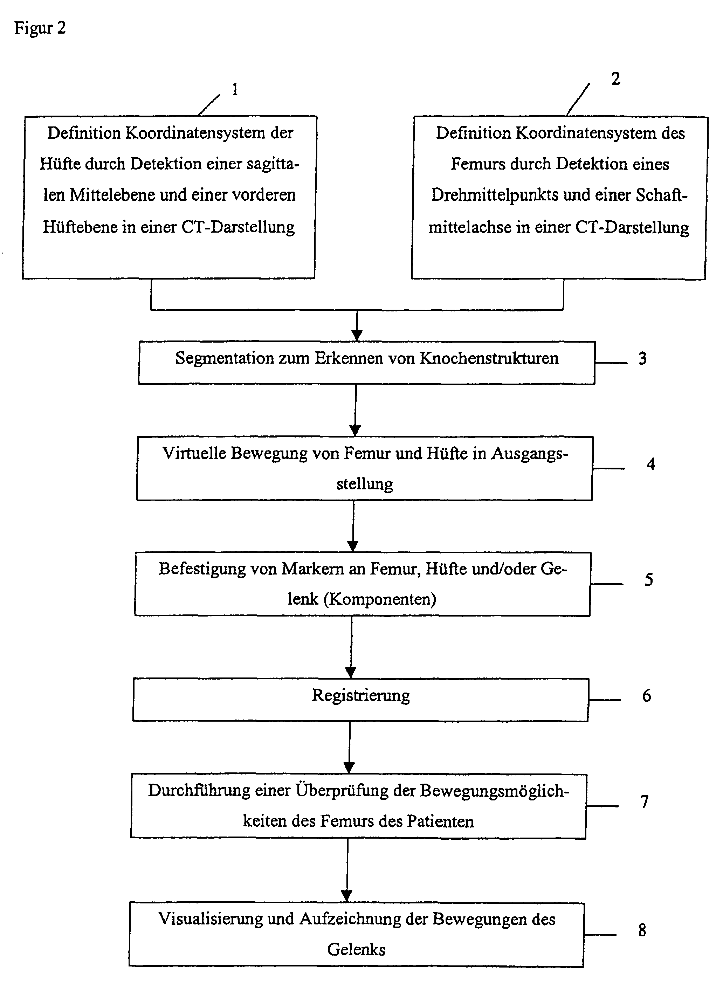

Figur 2 zeigt ein Ablaufdiagramm für eine Ausführungsform des erfindungsgemäßen Verfahrens. In Schritt 1 wird das Koordinatensystem der Hüfte definiert durch die Erkennung einer sagittalen Mittelebene und vorderen Hüftebene in einer Computertomographie(CT)-Darstellung. In Schritt 2 wird das Koordinatensystem des Oberschenkelknochens (Femur) definiert durch Erkennen eines Drehmittelpunktes und einer Mittelachse in einer Computertomographie-Darstellung. Die Schritte 1 und 2 können sowohl gleichzeitig als auch in beliebiger Reihenfolge hintereinander ausgeführt werden, d. h. Schritt 2 kann zum Beispiel auch vor Schritt 1 ausgeführt werden.

In Schritt 3 wird eine Segmentation durchgeführt, um in den Aufnahmen einer Körperstruktur die einzelnen Knochenstrukturen, wie zum Beispiel Hüfte, rechter Oberschenkelknochen und linker Oberschenkelknochen unterscheiden zu können.

Anschließend wird in Schritt 4 durch Simulation eine virtuelle Bewegung eines oder beider Oberschenkelknochen und der Hüfte zu einer Neutral- oder Ausgangsposition simuliert, wobei in einer Ausgangsposition beispielsweise die Koronalebene des Oberschenkelknochens parallel zur vorderen Ebene der Hüfte liegt, um eine Nullstellung zu definieren.

In Schritt 5 werden Marker, wie zum Beispiel Referenzsterne, an dem Oberschenkelknochen und der Hüfte und/oder an der Gelenkpfanne und dem Gelenkkopf der zu behandelnden Seite angebracht.

Anschließend werden in Schritt 6 die jeweiligen Elemente registriert.

In Schritt 7 wird eine Überprüfung der Bewegungsmöglichkeiten bzw. des Bewegungsbereichs des Oberschenkelknochens eines Patienten durchgeführt.

Anschließend werden in Schritt 8 zum Beispiel die maximal möglichen Bewegungen, ausgedrückt durch Öffnungswinkel des Gelenks in verschiedene Richtungen, ermittelt, visualisiert und aufgezeichnet. Dabei kann eine Abduktion (Abspreizen bzw. Bewegung des Gelenks nach hinten), Adduktion (Anführen bzw. Bewegung des Gelenks nach vorne), Flexion (Beugung) und/oder Extension (Streckung) des Hüftgelenks oder eines anderen Gelenks eines Patienten durchgeführt werden. Es ist auch möglich die möglichen Bewegungen mit den Bewegungsmöglichkeiten eines noch gesunden Gelenks des Patienten zu vergleichen.

Optional kann die Überprüfung der Bewegungsmöglichkeiten bzw. des Bewegungsbereichs des Oberschenkelknochens eines Patienten durch einen Vergleich mit den Bewegungsmöglichkeiten bzw. dem Bewegungsbereich eines symmetrisch dazu liegenden gesunden Gelenks durchgeführt werden.

Die Bewegung des Gelenks kann sowohl manuell, als auch automatisch zum Beispiel unter Verwendung eines Roboters durchgeführt werden, um definierte Kräfte an das Gelenk in definierten Richtungen anzulegen.

Obwohl die Erfindung beispielhaft anhand eines Hüftgelenks beschrieben wurde, ist es offensichtlich, dass die erfindungsgemäße Vorrichtung und das erfindungsgemäße Verfahren auch zum Ermitteln von Öffnungswinkeln anderer Gelenke, wie zum Beispiel eines Kniegelenks oder eines Armgelenks verwendet werden kann.

## Patentansprüche

1. Vorrichtung zum Bestimmen des Öffnungswinkels eines Gelenks (1, 2) mit einer Recheneinheit (5) zur Ermittlung des Öffnungswinkels des Gelenks (1, 2), **dadurch gekennzeichnet, daß** die Vorrichtung eine Erfassungsvorrichtung (4) umfaβt, welche die Position der Gelenkkomponenten (1, 2) und/oder die Position von mit dem Gelenk (1, 2) verbundenen oder zu verbindenden Strukturen erfassen kann.

2. Vorrichtung nach Anspruch 1 mit einer Speichereinheit (6) zum Speichern der geometrischen Struktur des Gelenks (1, 2) und/oder zum Speichern von Referenzwerten für die Messung des Öffnungswinkels.

3. Vorrichtung nach einem der vorhergehenden Ansprüche mit einer Datenausgabevorrichtung (7) zur Ausgabe des ermittelten Öffnungswinkels.

4. Vorrichtung nach einem der vorhergehenden Ansprüche mit einem Roboter und/oder einer Kraftmessvorrichtung zum Anlegen von definierten Kräften in definierten Richtungen an das Gelenk (1, 2).

5. Verfahren zum Bestimmen des Öffnungswinkels eines Gelenks (1, 2), wobei die Position der das Gelenk bildenden Komponenten (1, 2) und/oder die Position von mit dem Gelenk verbundenen oder zu verbindenden Strukturen mittels einer Erfassungsvorrichtung (4) erfasst und aus den erfassten Positionsdaten der Öffnungswinkels des Gelenks (1, 2) ermittelt wird.

6. Verfahren nach dem vorhergehenden Anspruch, wobei Aufnahmen des Gelenks und/oder der mit dem Gelenk verbundenen oder zu verbindenden Strukturen zur Bestimmung des Öffnungswinkels verwendet werden.

7. Verfahren nach dem vorhergehenden Anspruch, wobei eine Segmentation zur Unterteilung der aufgenommenen Strukturen durchgeführt wird.

8. Verfahren nach einem der drei vorhergehenden Ansprüche, wobei Marker (3a) an dem Gelenk (1, 2) und/oder an der mit dem Gelenk (1, 2) verbundenen oder zu verbindenden Strukturen angebracht werden.

9. Verfahren nach dem vorhergehenden Anspruch, wobei die einzelnen Komponenten des Gelenks (1, 2) und/oder die mit dem Gelenk verbundenen oder zu verbindenden Strukturen registriert werden.

10. Verfahren nach einem der fünf vorhergehenden Ansprüche, wobei der ermittelte Öff nungswinkel visualisiert wird.

11. Verfahren nach einem der sechs vorhergehenden Ansprüche, wobei gemessene Öff nungswinkel in einer Speichereinheit (6) gespeichert werden.

12. Verfahren nach einem der sieben vorhergehenden Ansprüche, wobei eine Messung von Öffnungswinkeln eines natürlichen Gelenks in verschiedenen Richtungen vor einer Implantation eines künstlichen Gelenks (1, 2) durchgeführt wird und die gemessenen Öffnungswinkel mit gemessenen Öffnungswinkeln eines implantierten künstlichen Gelenks (1, 2) verglichen werden.

13. Verfahren nach einem der acht vorhergehenden Ansprüche, wobei definierte Kräfte in definierten Richtungen an das Gelenk (1, 2) angelegt werden.

14. Computerprogramm, welches wenn es in einen Computer geladen ist oder auf einem Computer läuft, die Verfahrensschritte nach einem der Ansprüche 5 bis 13 durchführt.

15. Speichermedium für ein Programm oder Computerprogrammprodukt,welches Speichermedium das Computerprogramm nach Anspruch 14 speichert.

## Claims

1. A device for determining the aperture angle of a joint (1, 2), comprising a computational unit (5) for ascertaining the aperture angle of the joint (1, 2), **characterised in that** said device comprises a detection device (4) which can detect the position of the joint components (1, 2) and/or the position of structures connected to or to be connected to the joint (1, 2).

2. The device as set forth in claim 1, comprising a storage unit (6) for storing the geometric structure of the joint (1, 2) and/or for storing reference values for measuring the aperture angle.

3. The device as set forth in any one of the preceding claims, comprising a data output device (7) for outputting the ascertained aperture angle.

4. The device as set forth in any one of the preceding claims, comprising a robot and/or a force measuring device for applying defined forces in defined directions onto the joint (1, 2).

5. A method for determining the aperture angle of a joint (1, 2), wherein the position of the components (1, 2) forming the joint and/or the position of structures connected to or to be connected to the joint is detected by means of a detection device (4) and the aperture angle of the joint (1, 2) is ascertained from the detected positional data.

6. The method as set forth in the preceding claim, wherein recordings of the joint and/or of the structures connected to or to be connected to the joint are used to determine the aperture angle.

7. The method as set forth in the preceding claim, wherein segmentation is performed, to sub-divide the recorded structures.

8. The method as set forth in any one of the preceding three claims, wherein markers (3a) are attached to the joint (1, 2) and/or to the structures connected to or to be connected to the joint (1, 2).

9. The method as set forth in the preceding claim, wherein the individual components of the joint (1, 2) and/or the structures connected to or to be connected to the joint are registered.

10. The method as set forth in any one of the preceding five claims, wherein the ascertained aperture angle is visualised.

11. The method as set forth in any one of the preceding six claims, wherein measured aperture angles are stored in a storage unit (6).

12. The method as set forth in any one of the preceding seven claims, wherein aperture angles of a natural joint are measured in various directions before implanting an artificial joint (1, 2) and the measured aperture angles are compared with measured aperture angles of an implanted joint (1, 2).

13. The method as set forth in any one of the preceding eight claims, wherein defined forces are applied in defined directions to the joint (1, 2).

14. A computer program which, when it is loaded onto a computer or run on a computer, performs the method steps as set forth in any one of claims 5 to 13.

15. A storage medium for a program or a computer program product, said storage medium storing the computer program as set forth in claim 14.

## Revendications

1. Dispositif de détermination de l'angle d'ouverture d'une, articulation (1, 2), qui comporte une unité de calcul (5) qui détermine l'angle d'ouverture de l'articulation (1, 2), **caractérisé en ce que** le dispositif comprend un dispositif de détection (4) qui peut déterminer la position des composants de l'articulation (1, 2) et/ou la position de structures reliées ou à relier à l'articulation (1, 2).

2. Dispositif selon la revendication 1, doté d'une unité de mémoire (6) qui conserve en mémoire la structure géométrique de l'articulation (1, 2) et/ou qui conserve en mémoire des valeurs de référence qui permettent la mesure de l'angle d'ouverture.

3. Dispositif selon l'une des revendications précédentes, doté d'un dispositif (7) qui fournit des données qui délivrent l'angle d'ouverture déterminé.

4. Dispositif selon l'une des revendications précédentes, doté d'un robot et/ou d'un dispositif de mesure de force pour appliquer sur l'articulation (1, 2) des forces définies dans des directions définies.

5. Procédé de détermination de l'angle d'ouverture d'une articulation (1, 2), la position des composants (1, 2) qui forment l'articulation et/ou la position de structures reliées ou à relier à l'articulation étant déterminées au moyen d'un dispositif de détection (4), l'angle d'ouverture de l'articulation (1, 2) étant déterminé à partir des données de position détectées.

6. Procédé selon l'une des revendications précédentes,
dans lequel des enregistrements de l'articulation et/où de structures reliées ou à relier à l'articulation sont utilisés pour déterminer l'angle d'ouverture.

7. Procédé selon la revendication précédente, dans lequel une segmentation est exécutée pour distinguer les structures enregistrées.

8. Procédé selon l'une des trois revendications qui précèdent, dans lequel des repères (3a) sont installés sur l'articulation (1, 2) ou sur les structures reliées ou à relier à l'articulation (1, 2).

9. Procédé selon la revendication précédente, dans lequel les composants individuels de l'articulation (1, 2) et/ou les structures reliées ou à relier à l'articulation sont enregistrés.

10. Procédé selon l'une des cinq revendications qui précèdent, dans lequel l'angle d'ouverture déterminé est visualisé.

11. Procédé selon l'une des six revendications qui précèdent, dans lequel l'angle d'ouverture mesuré est conservé en mémoire dans une unité de mémoire (6).

12. Procédé selon l'une des sept revendications qui précèdent, dans lequel une mesure des angles d'ouverture d'une articulation naturelle est réalisée dans différentes directions avant l'implantation d'une articulation artificielle (1, 2), les angles d'ouverture mesurés étant comparés à des angles d'ouverture mesurés sur une articulation artificielle (1, 2) qui a été implantée.

13. Procédé selon l'une des huit revendications qui précèdent, dans lequel des forces définies sont appliquées sur l'articulation (1, 2) dans des directions définies.

14. Programme informatique qui, lorsqu'il a été chargé dans un ordinateur ou est exécuté sur un ordinateur, exécute les étapes de procédé selon l'une des revendications 5 à 13.

15. Support de mémoire pour un programme ou un produit de programme informatique, lequel support de mémoire conserve le programme informatique selon la revendication 14.
